(19) Europäisches Patentamt
European Patent Office
Office européen des brevets

(11) **EP 4 400 871 A1**

(12) **EUROPEAN PATENT APPLICATION**

(43) Date of publication:
**17.07.2024 Bulletin 2024/29**

(21) Application number: **23151344.1**

(22) Date of filing: **12.01.2023**

(51) International Patent Classification (IPC):
**G01T 1/161** (2006.01)    **G01T 1/29** (2006.01)
**A61B 6/03** (2006.01)

(52) Cooperative Patent Classification (CPC):
**G01T 1/1615; A61B 6/037; A61B 6/4258;
A61B 6/4266; A61B 6/4429; G01T 1/2985**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Universiteit Gent
9000 Gent (BE)**

(72) Inventor: **VANDENBERGHE, Stefaan
9860 Scheldewindeke (BE)**

(74) Representative: **De Clercq & Partners
Edgard Gevaertdreef 10a
9830 Sint-Martens-Latem (BE)**

(54) **POSITRON EMISSION TOMOGRAPHY SYSTEM AND METHOD**

(57)    The present disclosure relates to the field of positron emission tomography imaging and, more in particular, to a method for PET imaging a target volume of a subject injected with a radiotracer, and a system configured for the same. The system for positron emission tomography (PET) imaging a target volume of a subject injected with a radiotracer may comprise at least two scanning modules, arranged opposite of each other and spaced apart so as to form a scanning area between them; wherein each of the at least two scanning modules comprises: at least one PET detector configured to detect the occurrence of a coincidence event in the scanning area; and, at least one radiation source configured to emit a gamma radiation that illuminates at least one PET detector of the opposite scanning module; and, a control unit in communication with the at least two scanning modules, that is configured to: acquire combined PET data related to the simultaneous detection of coincidence events from the PET detectors; spatially separate the combined PET data into transmission data and emission data; reconstruct a transmission image from the transmission data, wherein the transmission image comprises anatomical information of the target volume; reconstruct an emission image from the emission data, wherein the emission image comprises molecular information of the radiotracer; and, generate a combined image based on the transmission image and the emission image.

**Description**

**FIELD OF THE INVENTION**

The present disclosure relates to the field of positron emission tomography imaging and, more in particular, to a method for PET imaging a target volume of a subject injected with a radiotracer, and a system configured for the same.

**BACKGROUND**

Combined positron emission tomography (PET) / computed tomography (CT) scanners have changed the paradigm of medical imaging due to their capability to combine molecular and anatomical information that allows for more accurate diagnosis than if the two scans were performed separately. PET/CT scanners are applied in various research studies, for example, dynamic imaging, low dose imaging, delayed imaging, brain-body interactions, new tracer development, and so on.

The high acquisition and service costs, however, are a major obstacle for clinical routine imaging in standard (nuclear) medical centres. A typical PET/CT scanner requires more detectors than a conventional PET, which is accompanied by a far higher number of events processed by the coincidence electronics at far higher rates. Sufficient computing power and memory should therefore be provided. As such, the complexity and cost of a single PET/CT scanner is far greater than a standalone PET scanner.

The number of clinical PET scans is nevertheless ever increasing, and it can be expected that there will be even more medical imaging required in the future from selected screening, for example, based on blood, genetic tests or patient history. PET imaging has thus an essential role in screening, (expensive) therapy prediction and follow-up studies. For example, early detection of a tumour may result in improved therapy outcomes and potentially follow-up scans in the future. Repeated imaging after screening (longitudinal studies) can be expected for various forms of cancer.

To satisfy the clinical needs without further increasing healthcare (imaging) costs, medical centres will need to be able to scan more patients. Increased patient throughput can be achieved with faster imaging (acquisition) times, and ideally this can be realized with lower cost imaging systems and with less personnel (often a limiting factor in medical centres). Finally, as there is a shift towards a healthier population (especially screening), there is a rationale towards lower dose imaging, which can in turn justify repeated scans for the same patient.

PET/CT scanners suffer from quite low effective patient throughput. One of the limiting factors is the time required for obtaining and processing a scout view and CT scan. CT scanning has become standard practise during PET/CT acquisition as this is an easy and fast way to obtain anatomical information about the patient for co-registration of PET data. However, because the PET and CT scans need to be performed in sequential mode, this increases the total scanning time per session. On the other hand, a standalone PET scanner lacks the anatomical information required to obtain the combined imaging mode

Another limiting factor is the patient positioning on the CT scanner table. It has become standard design for a PET/CT scanner to include a flat table for the patient to lie on their back that passes into the CT scanner. However, the time required to assist a patient, who commonly feels anxious or claustrophobic, onto the scanning table greatly impacts the scanner downtime between successive patient scans. Hence, there is a need to remedy the limitations of current PET/CT protocols by providing for a more efficient imaging system that is capable of higher patient throughput whilst still providing the combined imaging mode generated by a PET/CT scanner.

**SUMMARY OF THE INVENTION**

As described above, there is a need to remedy the limitations of combined positron emission tomography (PET) / computed tomography (CT) systems. PET/CT scanners are an important diagnostic tool for imaging the dynamic radiopharmaceutical distributions in the major organs of the body. Given the need for high patient throughput, patient scan time efficiency is a challenge.

The innovation of the herein disclosed technology is based on the observation that a key limiting factor in a PET/CT scanner is the CT scanning time and/or the subject positioning. Based on this observation, the disclosed technology aims to steers the PET imaging process towards a, preferably simultaneous, acquisition of anatomical and molecular information to generate an image that is similar to a combined PET/CT image. This approach can be applied for imaging of a specific target volume of a subject, which may include the total body or a selected portion thereof.

Hence, compared to the "multi-step" approach of a combined PET/CT scanner, in which the relevant PET and CT data is sequentially acquired and processed by the respective PET and CT scanners, the present disclosure describes a "unified" approach based on a modified PET scanning protocol that is capable of reducing the total scanning time per subject. That is, the time required for acquiring a CT scan can be effectively omitted without losing the anatomical information that has become a standard for improving the accuracy of diagnosis in medical imaging. This PET scanner design can be realised with a smaller detector surface that shows comparable sensitivity to PET/CT scanners at a reduced complexity, and hence cost.

Additionally, based on this modified PET scanning protocol, the design of the PET scanner system can be adapted to depart from the classical patient positioning on a scanner table by allowing a subject to be scanned in an upright position (for example, while standing or sitting) between a pair of oppositely arranged, preferably flat, detector modules. This modified PET scanner design can be implemented as a "walk-through" PET scanner that can enable a more subject driven PET scanning procedure to reduce the length of waiting queues at medical centres and alleviate healthcare workers workload.

A first overview of various aspects of the technology according to the present disclosure is given hereinbelow, after which specific embodiments will be described in more detail. This first overview is meant to aid the reader in understanding the technological concepts more quickly, but it is not meant to identify the most important or essential features thereof, nor is it meant to limit the scope of the present disclosure.

An aspect of the present disclosure relates to a (computer-implemented) method for PET imaging a target volume of a subject injected with a radiotracer, the method comprising the steps of:

- providing a PET system comprising at least two scanning modules, arranged opposite of each other, and spaced apart so as to form a scanning area between them; wherein each of the at least two scanning modules comprises at least one PET detector configured to detect the occurrence of a coincidence event in the scanning area, and at least one radiation source configured to emit gamma radiation that illuminates at least one PET detector of the opposite scanning module;
- acquiring combined PET data related to the simultaneous detection of coincidence events;
- spatially separating the combined PET data into transmission data and emission data;
- reconstructing a transmission image from the transmission data, that comprises anatomical information of the target volume;
- reconstructing an emission image from the emission data, that comprises molecular information of the radiotracer; and
- generating a combined image based on the transmission image and the emission image.

A further aspect of the present disclosure relates to a system for PET imaging a target volume of a subject injected with a radiotracer, the system comprising

- at least two scanning modules, arranged opposite of each other and spaced apart so as to form a scanning area between them; wherein each of the at least two scanning modules comprises: at least one PET detector configured to detect the occurrence of a coincidence event in the scanning area; and, at least one radiation source configured to emit a gamma radiation that illuminates at least one PET detector of the opposite scanning module; and,

- a control unit in communication with the at least two scanning modules, that is configured to:
- acquire combined PET data related to the simultaneous detection of coincidence events from the PET detectors;
- spatially separate the combined PET data into transmission data and emission data;
- reconstruct a transmission image from the transmission data, that comprises anatomical information of the target volume;
- reconstruct an emission image from the emission data, that comprises molecular information of the radiotracer; and,
- generate a combined image based on the transmission image and the emission image.

In some embodiments the combined PET data is separated based on a time of flight (TOF) profile of the detected coincidence events; preferably by determining the time difference between detection of two photons forming the detected coincidence event and determining a position of an emission point based on said time difference.

In some embodiments the combined PET data is separated based on an energy profile of the detected coincidence events.

In some embodiments the transmission data is transformed by applying an inverse bilinear transformation so that the transmission image resembles an image obtained by a lower energy scan, preferably resembles a lower energy computerized tomography (CT) scan.

In some embodiments the transmission image reconstructed from the transmission data and a blank data that is acquired by performing a scan with the subject absent.

In some embodiments the detector module and/or PET detector are an essentially flat panel.

In some embodiments a gap between the opposite detector modules is at least 40 cm to at most 100 cm, preferably 40 cm to 80 cm, more preferably 40 cm to 70 cm, more preferably still 45 cm to 60 cm, more preferably still 50 cm to 55 cm.

In some embodiments the detector modules are moveably arranged onto a support member so that their position can be adjusted based on a height of the subject.

In some embodiments the radiation source is moveably arranged in the detector module (10) so that it can be moved over the PET detector during scanning.

In some embodiments the radiation source is spaced apart from the PET detector by a gap of at least 0.1 cm to at most 10.0 cm, preferably 0.5 cm to at most 5.0 cm; more preferably 1.0 cm to 4.0 cm, more preferably still 2.0 cm to 3.0 cm.

In some embodiments the PET detector comprises an array of a pixelated detector, preferably, LYSO, CdW04, Csl(TI), BGO, and/or other materials such as scintillating plastic.

In some embodiments the PET detector comprises an array of monolithic scintillation crystals; preferably lutetium-yttrium oxyorthosilicate (LYSO) and/or bismuth germanate (BGO) crystals.

In some embodiments the system is a walk-through scanner configured to scan the subject in an upright position.

In some embodiments the system comprises a means for positioning the subject in an in in an upright position in the PET scanning area; preferably comprising a handlebar that can be gripped by the subject, a foot stand that the subject can stand on and/or a chair that the subject can sit on.

## DESCRIPTION OF THE FIGURES

The following description of the figures relate to specific embodiments of the disclosure which are merely exemplary in nature and not intended to limit the present teachings, their application or uses.

Throughout the drawings, the corresponding reference numerals indicate the following parts and features: PET scanner (1); scanning module (10); PET detector (11); radiation source (12); handlebar (20) foot stand (21).

> **Figure 1** shows a schematic representation of a method for PET imaging a target volume of a subject according to a preferred embodiment of the present disclosure.
> **Figure 2** shows a cross-section of a PET scanner system 1 for PET imaging a target volume of a subject according to a preferred embodiment of the present disclosure.
> **Figure 3** is an illustration of walkthrough PET scanner according to a preferred embodiment of the present disclosure.
> **Figure 4** is a close-up of a scanning module (10) of the PET scanner of **Figure 3.**
> **Figure 5** is an image of the walkthrough PET scanner of **Figure 3.**
> **Figure 6** shows how the photons from an emission coincidence event can be detected.
> **Figure 7** shows how the photons from a transmission coincidence event can be detected.
> **Figure 8** shows how a transmission image obtained at a higher energy scan can be transformed to resembles an image obtained by a lower energy CT scan, for example, by applying an inverse bilinear transformation.

## DETAILED DESCRIPTION

In the following detailed description, the technology underlying the present disclosure will be described in the form of various technological aspects. It will be readily understood that the aspects of the present disclosure, as generally described herein, and illustrated in the figures, can be arranged, substituted, combined, and designed in a wide variety of different configurations, all of which are explicitly contemplated and make part of this disclosure. This description is meant to aid the reader in understanding the technological concepts more easily, but it is not meant to limit the scope of the present disclosure, which is limited only by the claims.

Reference throughout this specification to "one embodiment" or "an embodiment" means that a particular feature, structure or characteristic described in connection with the embodiment is included in at least one embodiment of the present disclosure. Thus, appearances of the phrases "in one embodiment" or "in an embodiment" in various places throughout this specification are not necessarily all referring to the same embodiment.

As used herein, the terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps. The singular forms "a", "an", and "the" include both singular and plural referents unless the context clearly dictates otherwise.

As used herein, relative terms, such as "left," "right," "front," "back," "top," "bottom," "over," "under," and the like, are used for descriptive purposes and not necessarily for describing permanent relative positions. It is to be understood that such terms are interchangeable under appropriate circumstances and that the embodiment as described herein are capable of operation in other orientations than those illustrated or described herein unless the context clearly dictates otherwise.

Objects described herein as being "adjacent" to each other reflect a functional relationship between the described objects, that is, the term indicates the described objects must be adjacent in a way to perform a designated function which may be a direct (i.e. physical) or indirect (i.e. close to or near) contact, as appropriate for the context in which the phrase is used.

Objects described herein as being "connected" or "coupled" reflect a functional relationship between the described objects, that is, the terms indicate the described objects must be connected in a way to perform a designated function which may be a direct or indirect connection in an electrical or nonelectrical (i.e. physical) manner, as appropriate for the context in which the term is used.

As used herein, the term "substantially" refers to the complete or nearly complete extent or degree of an action, characteristic, property, state, structure, item, or result. For example, an object that is "substantially" enclosed would mean that the object is either completely enclosed or nearly completely enclosed. The exact allowable degree of deviation from absolute completeness may in some cases depend on the specific context. However-

ever, generally speaking the nearness of completion will be so as to have the same overall result as if absolute and total completion were obtained. The use of "substantially" is equally applicable when used in a negative connotation to refer to the complete or near complete lack of an action, characteristic, property, state, structure, item, or result.

As used herein, the term "about" is used to provide flexibility to a numerical value or range endpoint by providing that a given value may be "a little above" or "a little below" said value or endpoint, depending on the specific context. Unless otherwise stated, use of the term "about" in accordance with a specific number or numerical range should also be understood to provide support for such numerical terms or range without the term "about". For example, the recitation of "about 30" should be construed as not only providing support for values a little above and a little below 30, but also for the actual numerical value of 30 as well.

The recitation of numerical ranges by endpoints includes all numbers and fractions subsumed within the respective ranges, as well as the recited endpoints. Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the disclosure described herein are capable of operation in other sequences than described or illustrated herein.

Reference in this specification may be made to devices, structures, systems, or methods that provide "improved" performance (e.g. increased or decreased results, depending on the context). It is to be understood that unless otherwise stated, such "improvement" is a measure of a benefit obtained based on a comparison to devices, structures, systems or methods in the prior art. Furthermore, it is to be understood that the degree of improved performance may vary between disclosed embodiments and that no equality or consistency in the amount, degree, or realization of improved performance is to be assumed as universally applicable.

In addition, embodiments of the present disclosure may include hardware, software, and electronic components or modules that, for purposes of discussion, may be illustrated and described as if the majority of the components were implemented solely in hardware. However, one of ordinary skill in the art, and based on a reading of this detailed description, would recognize that, in at least one embodiment, the electronic based aspects of the present disclosure may be implemented in software (e.g., instructions stored on non-transitory computer-readable medium) executable by one or more processing units, such as a microprocessor and/or application specific integrated circuits. As such, it should be noted that a plurality of hardware and software-based devices, as well as a plurality of different structural components may be utilized to implement the technology of the present disclosure. For example, "servers" and "computing devices" described in the specification can include one or more processing units, one or more computer-readable medium modules, one or more input/output interfaces, and various connections connecting the components.

In the present description, technology is described by means of which the efficiency of a PET imaging workflow can be improved. PET is an important tool for (medical) imaging of a target region of a subject. As used herein, the subject may refer to a human subject, for example, a healthy individual or a patient suffering from a specific pathology. Alternatively, the subject can be nonhuman, for example, an animal subject.

Imaging through Positron emission tomography (PET) techniques is well known and has been widely applied in (medical) imaging applications. In essence, a typical PET technique acquires (medical) imaging data by using a radionuclide that is injected together with a tracer compound into the subject under examination. After injection, the radionuclide starts decaying leading to emission of positrons and the resulting positrons will subsequently annihilate on contact with electrons after travelling a specific distance within the subject's body. Each annihilation produces two photons travelling in opposite directions that can be detected by a detector capable of registering incident gamma rays.

The emission of these photons is measured by acquisition of a "emission scan" when the subject under examination is placed in the scanner within the field of view (FOV) of oppositely arranged detectors after the radiotracer injection. The detector electronics are linked so that two detection events occurring within a certain time window may be called coincident and thus be determined to have come from the same annihilation. These coincidence events can be stored, for example, as raw image data, list mode data or sinograms corresponding to projections within a specific target region of the subject. An image can then be reconstructed from the acquired data using standard tomographic techniques known in the art, for example, to show the tracer distribution throughout the target region.

The two photons must be detected during predetermined time window to be counted as a true coincidence event. Most of the photons are, however, lost because of their absorption in the body. This loss of detection of true coincidence events is called "attenuation" and it can result in artifacts in the reconstructed image unless it is corrected during reconstruction. The attenuation can be measured by acquisition of a separate "transmission scan" when the subject under examination is placed in the scanner before the radiotracer injection. The transmission scan can be performed using an external radioactive positron-emitting source. For reference, another scan with the same source may be performed at another time (before or after) without the subject present in the scanning area (blank data). A blank scan followed by transmission scan for all possible coincidence lines therefore enables measurement of transmission count rates. Attenuation correction factors can then be computed from the transmission and blank scans. As previously described, a combined positron emission tomography (PET) / computed tomography (CT) imaging framework consist of a "multi-step" approach. First, image data is sequentially acquired by the respective PET and CT devices, after which the images are individually reconstructed from the acquired PET/ CT data. Next, the reconstructed images are mutually geometrically aligned. Finally, an aligned image is generated comprising the relevant anatomical and molecular information.

The innovation of the herein disclosed technology is based on the observation that one of the limiting factors in a PET/CT system is the CT scanning time and/or the subject positioning. Based on this observation, the disclosed technology aims to steers the PET imaging process towards a simultaneous acquisition of anatomical and molecular information such that an image can be generated in which the relevant is combined in a way that is similar to an aligned PET/CT image.

As used herein "simultaneous" with reference to the PET scanning procedure means that at least a portion of the PET image data corresponding the emission and transmission scans is acquired at the same time, as opposed to the image data being acquired sequentially. Besides the self-explanatory timesaving from reducing the total scanning time, this is moreover advantageous because there is no specific alignment required as the emission and transmission images are obtained simultaneously. Hence, the complexity of post-reconstruction image processing can be reduced.

Consequently, the present disclosure describes a "unified" approach based on a modified PET scanning protocol that is capable of reducing the total scanning time per subject at a reduced complexity, and hence cost. That is, the time and resources needed for obtaining a scout and CT scan can be effectively omitted without losing the anatomical information that has become a standard in medical imaging for improve the quality of diagnosis.

Additionally, based on this modified PET scanning protocol, the design of the PET scanner system can be adapted to departs from the classical patient positioning on a scanner table by allowing a subject to be scanned in an upright position (for example, while standing or sitting) between a pair of oppositely arranged, preferably flat, detector modules. This modified PET scanner design can be implemented as a "walk-through" PET scanner that can enable a more subject driven PET scanning procedure to reduce the length of waiting queues at medical centres and alleviate healthcare workers workload.

The herein disclosed technology can be regarded as "general purpose" (medical) PET imaging technology in the sense that it can be readily adapted for imaging of various and different target volumes. As used herein, the "target volume" of a subject may refer to any portion of the subject's body for which PET data can be acquired by PET scanning, which may include, for example, one or more organs or body parts. In a preferred embodiment, the target volume may comprise the subject's total body or the subject's torso (typically the lower body is lesser importance than the torso containing the organs). The skilled person will, hence, appreciate that the disclosed technology is not limited to a specific anatomy or pathology, provided that the target volume can be inspected using PET technology, i.e., that PET data can be acquired of the target volume.

Therefore, in effect, by way of the technology described herein, it is possible to use a (computer implemented) technique in a unified approach that directly generates an image comprising both the relevant molecular and anatomical information of a target volume, referred to herein as a "density map". Specifically, it is possible to generate (in a virtual way) a density map in which the relevant molecular and anatomical information are geometrically aligned based on a simultaneous acquisition of emission image data, that is, based on simultaneously performed emission and transmission scans.

An overview of various aspects of the technology of the present disclosure is given hereinbelow, after which specific embodiments will be described in more detail. This overview is meant to aid the reader in understanding the technological concepts more quickly, but it is not meant to identify the most important or essential features thereof, nor is it meant to limit the scope of the present disclosure. When describing specific embodiments, reference is made to the accompanying drawings, which are provided solely to aid in the understanding of the described embodiment.

An aspect of the present disclosure relates to a (computer implemented) method for PET imaging a target volume of a subject injected with a radiotracer, the method comprising the steps of:

- providing a PET system comprising at least two scanning modules, arranged opposite of each other, and spaced apart so as to form a scanning area between the scanning modules; wherein each of the at least two scanning modules comprises at least one imaging detector configured to detect the occurrence of a coincidence event in the scanning area, and at least one radiation source configured to emit gamma radiation that illuminates at least one imaging detector of the opposite scanning module;
- acquiring combined image data related to the simultaneous detection of one or more coincidence events;
- spatially separating the combined image data into transmission data and emission data;
- reconstructing a transmission image from the transmission data, that comprises anatomical information of the target volume;
- reconstructing an emission image from the emission data, that comprises molecular information of the radiotracer;

- generating a combined image based on the transmission image and the emission image.

Another aspect of the present disclosure relates to a system for PET imaging of a target volume of a subject injected with a radiotracer, the system comprising: at least two scanning modules, arranged opposite of each other and spaced apart so as to form a scanning area between the scanning modules; wherein each of the at least two scanning modules comprises at least one imaging detector configured to detect the occurrence of a coincidence event in the scanning area; and, at least one radiation source configured to emit gamma radiation that illuminates at least one imaging detector of the opposite scanning module; and, a control unit in communication with the at least two scanning modules, the control unit being configured to: acquire combined image data related to the simultaneous detection of one or more coincidence events from the imaging detectors; spatially separate the combined image data into transmission data and emission data; reconstruct a transmission image comprising anatomical information of the target volume from the transmission data; reconstruct an emission image comprising molecular information of the radiotracer from the emission data; and, generate a combined image based on the transmission image and the emission image.

It is understood, based on any of the embodiments described herein, that the herein disclosed method can be performed by the herein disclosed system. That is, the system can be configured in a way that it can perform any of the steps of the method. This configuration may include an adaptation to any of the hardware of the system, for example, the scanning modules, and may also include an adaptation to the control unit processing data received from the system's hardware, for example, the image data. In an example, if the method refers to an embodiment wherein, for example, the emission and transmission scans are performed simultaneously, the system may hence be configured in a way that the emission and transmission scans can performed simultaneously, for example, by synchronizing the components of the detector modules and the radiation source.

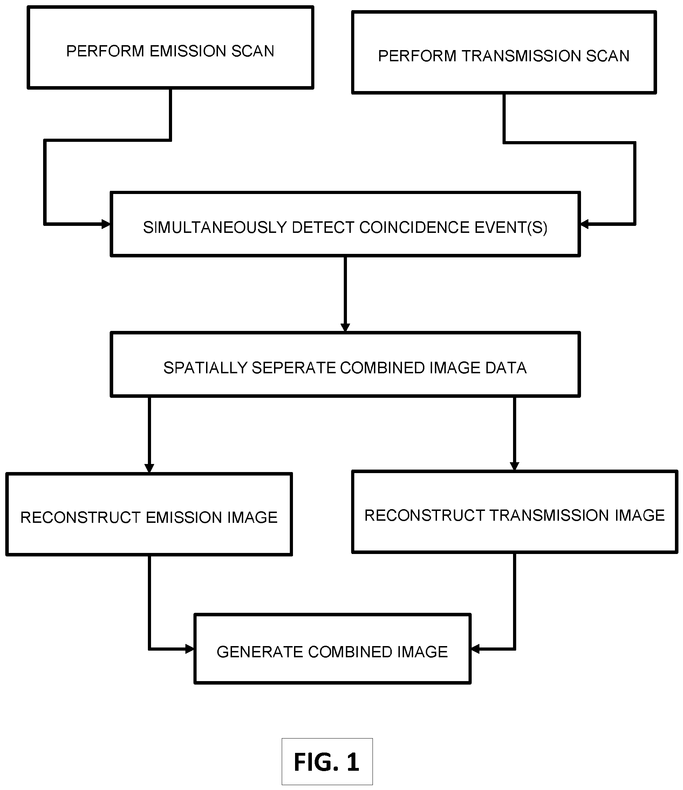

The present technology will be discussed in more detail with reference to **Figure 1,** which schematically illustrates a (computer implemented) method for PET imaging according to a preferred embodiment. As previously described, one of the limitations of a standalone PET scanner is that no direct measurement of a "density map" (attenuation of the subject) can be obtained in its conventional configuration, in which the anatomical and molecular information of the subject under examination is combined. As such, a modified PET scanning protocol is disclosed in which such a density map can be obtained by acquiring an additional transmission scan, that is, in addition to the emission scan that is performed in a conventional PET scanner.

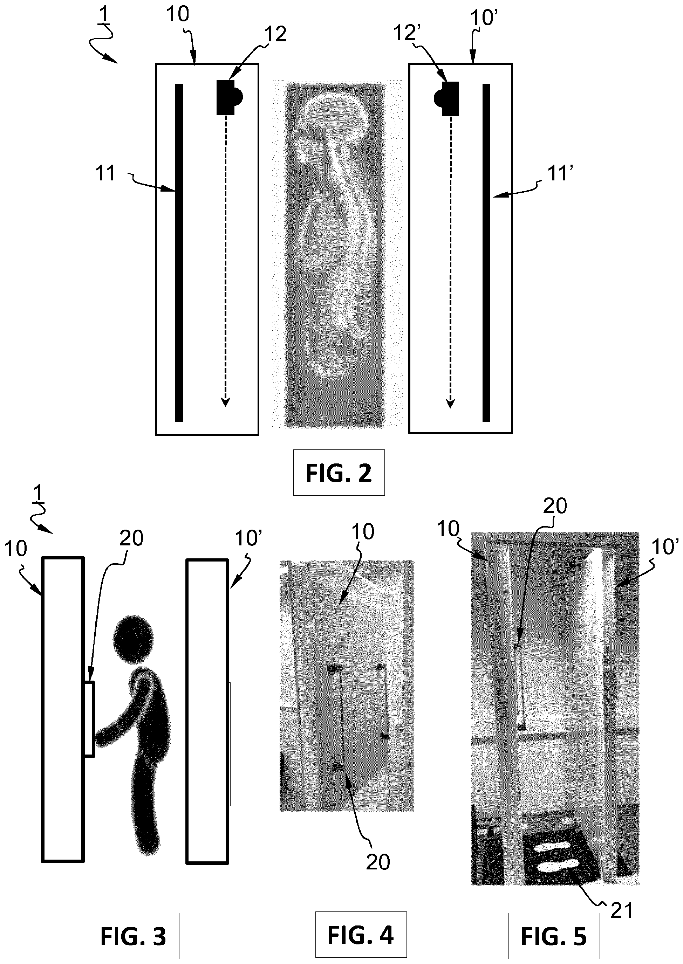

The transmission scan can be performed by providing a radiation source in the PET scanner. An exemplary preferred embodiment of a PET scanner system 1 comprising a radiation source 12 is shown in Figure 2. More specifically, the system 1 is shown to comprise a pair of two scanning modules 10 that are sufficiently spaced apart so that a scanning area is formed between them that is suitable for the positioning of the subject. Each scanning module is further shown to comprise a detector 11 and a radiation source 12 that is arranged in front of said detector 11 such that it is closer to the position of the subject. Advantageously, the radiation source 12 is moveably arranged so that it can move over the detector 11 during scanning - the movement is illustrated with a dashed downward arrow. Hereunder embodiments of the detector will be discussed first, and embodiments of the radiation source will be described later.

In some embodiments the detector may comprise any type of scintillating material known in the art that is suitable for detecting of emission coincidence events. Preferably, the detector may comprise an array of monolithic scintillation crystals, for example, lutetium-yttrium oxyorthosilicate (LYSO) and/or bismuth germanate (BGO) crystals. Alternatively or in combination, detector may comprise a pixelated detector, for example, LYSO, CdW04, CsI(TI), BGO, and/or other materials such as scintillating plastic. Nevertheless, a monolithic detector offers an improved spatial resolution and 3D spatial information that makes it more advantageous for the measurement of a density map. It is desired that the detector can deliver high isotropic spatial resolution in image space, preferably about 2 mm over the whole FOV.

In the embodiment of **Figure 2,** the detector 11 is illustrated as a continuous panel. However, those skilled in the art will understand that a detector panel commonly comprises an array of scintillating materials that are coupled so that they form a unified detector. In some embodiments, the arrays of the detector may have a dimension of at least $1\times1$ cm to at most $10\times10$ cm, preferably $2\times2$ cm to at most $9\times9$ cm, more preferably $3\times3$ cm to at most $8\times8$ cm, more preferably still $4\times4$ cm to $7\times7$ cm, more preferably still $5\times5$ cm to $6\times6$ cm. Although there is no actual limitation on the array dimension as far as the present application is concerned, the preferred dimensions have been selected based on practical issues encountered for producing monolithic blocks.

In some embodiments, the detector modules and/or detectors may comprise essentially flat panels, that is, the surface of the detector modules and/or detectors is essentially flat with low to preferably no curvature. Alternatively or in combination, the detector modules and/or detectors may comprise at least partially curved panels. Essentially flat panels are, however, advantageous for determining 3D spatial information because this allows for a continuous panel design contrary to curved panels for which one or more gaps may be expected in the set-up. Additionally, opposite flat panels allow for a PET scanner design that is easier to manoeuvre for the subject in an upright scanning position, such as in embodiments of a walkthrough PET scanner discussed later.

In some embodiments, the detector has thickness of at least 10 cm to at most 30 cm, preferably 11 cm to at most 20 cm, more preferably 12 cm to at most 19 cm, more preferably still 13 cm to 18 cm, more preferably still 14 cm to 17 cm, more preferably still 15 cm to 16 cm. Although there is no actual limitation on the detector thickness as far as the present application is concerned, the preferred dimensions have been selected based on a practical implementation for an upright standing scanner, as will be discussed later.

In some embodiments, the detector modules and/or detectors may be fixed onto a support member. The detector modules and/or detectors may have a length that is sufficient to scan the entire height of the subject such that a total body scan is possible. Advantageously, the panels are arranged so that the top of the panels is at least 10 cm higher than the top of the subject's head.

In some embodiments, the detector modules and/or detectors may be moveably arranged onto the support member so that their position can be adjusted based on the height of the subject. Alternatively or in combination, the subject may be moved relative to the detector modules and/or detectors, for example, by arranging a moveable platform between the detector modules and/or detectors. Optionally, the detector modules, detectors, and/or subject may be moved during the scanning process, for example, between two successive scans, such that a total body scan is possible even if the length of the detector modules and/or detectors does not match the subject's height.

In some embodiments, the detector modules and/or the detectors may be spaced apart by a gap of at least 40 cm to at most 100 cm, preferably 40 cm to 80 cm, more preferably 40 cm to 70 cm, more preferably still 45 cm to 60 cm, more preferably still 50 cm to 55 cm. Although there is no actual limitation on the detector thickness as far as the present application is concerned, the preferred dimensions have been selected based on a practical implementation for a subject in an upright position. More specifically, the preferred dimensions are selected such that the detector modules and/or the detectors are as close as possible to the subject's body. This proximity allows for improved scanning accuracy. It may hence be appreciated that the disclosed configuration allows for the panels to be much closer to the subject than, for example, in a PET/CT system, in which the gap between the detectors is typically 75-85 cm, that is, the diameter of a common gantry aperture.

Referring back again to **Figure 2,** it is shown that the radiation source 12 is moveably arranged such that it can be moved over the detector 11. For example, the radiation source 12 is shown to move up and down relative to the subject. Emissions from this source will lead to additional coincidences. The ratio of these data with the subject present and with the subject absent can allow for reconstruction of a transmission image comprising anatomical information of the subject.

In some embodiments, the radiation source may be spaced apart from the detector surface such that it can move over the detector without damaging it or causing interference. For example, there may a gap of at least 0.1 cm to at most 10.0 cm between the radiation source and the detector, more preferably least 1.0 cm to at most 5.0 cm, for example 2.0, 3.0 cm or 4.0 cm. The gap is preferably kept as small as possible to realise a compact design.

In some embodiments, the radiation source may comprise a long-lived positron emitting sources may be used, such as Ge-68/Ga-68. Preferably, a single photon emitter can be used to improve statistics and reduce scan time, such as Cs-137.

In any of the above embodiments, the transmission scan can be performed while simultaneously performing an emission scan, so that the coincidence events from an emission source and the events from a radiation source can be detected simultaneously. In this way, the simultaneous scan can deliver the required data for making the PET scan quantitative (attenuation correction).

As previously described, the present disclosure describes upright PET scanner system that allows a subject to be scanned in an upright position (for example, while standing or sitting). The upright design is particularly suitable for implementation as a "walk-through" PET scanner. An exemplary preferred embodiment of an upright PET scanner 1 is shown in **Figure 3,** which comprises the pair of oppositely arranged scanning modules 10 in accordance with any of the embodiments described above. Additionally, the upright PET scanner 1 may comprise a means for guiding the patient for the scanning procedure and assume a desired scanning position. For example, as further shown in **Figure 4,** the PET scanner 1 may comprise a handlebar 20 that can be gripped by the subject. In another example, as further shown in **Figure 5,** the PET scanner 1 may comprise a foot stand that the subject can stand on. Other means for enabling a subject driven scanning procedure may be provided, that is, performing the PET scanning procedure the need for assistance from healthcare workers to ensure proper positioning and advantageously completely independent scanning procedures. Such means will be described below.

In some embodiments the PET scanner may comprise a gripping member that can be gripped by the hands of the subject. For example, the gripping means may be a bar or rod that has a thickness which is suitable for gripping. The presence of a gripping means guides the subject towards the desired orientation between the detector modules and may also support the subject so that the upper body can kept stable. Optionally, the gripping means may comprise a sensor that registers it being gripped by the subject, as will be discussed later.

In some embodiments the PET scanner may comprise a standing means for the subject to stand on and/or a sitting means for the subject to sit on. For example, the standing member may be a pair of footprints.

For example, the sitting means may be chair or stool for the subject to sit on. The presence of a sitting/standing means guides the subject towards the desired scanning positions.

The system may comprise a control unit configured to detect the presence and/or positioning of the subject and control the PET scanner based on a predefined requirement, for example, by starting a PET scan when the subject is correctly positioned. For example, in one of the above embodiments, the gripping means may comprise a sensor that registers it being gripped by the subject. In another embodiment, the sitting/standing means may comprise a sensor that registers the presence of the subject, for example, a pressure sensor. Alternatively or in combination with any of the above embodiments, the system may comprise a motion detector and/or camera that register the movement of the subject.

In some embodiment, the system may comprise a control unit configured to track the breathing pattern of the subject and control the PET scan based on a predefined requirement, for example, by starting a PET scan when the subject does not move. Movements of the chest may impact the accuracy of the scan; hence, the scan may be started when the subject holds their breath.

In some embodiment, the system may be a walk-through system, preferably wherein the step of acquiring imaging data lasts at most 60 sec, preferably at most 45 sec, preferably at most 30 sec.

Returning back to the embodiment of **Figure 1,** once the coincidence event(s) have been detected and stored as combined image data, data corresponding to one or more coincidence events caused by an emission of the radiotracer, referred to hereinbelow as "emission data", and corresponding to one or more coincidence events caused by an emission of the radioactive source, referred to hereinbelow as "transmission data", can be spatially separated.

To separate the transmission data from the emission data, knowledge of where each coincidence of the photon-pair was created can be used. In a perfect PET system, for each measured coincidence the difference in detection times between the two photons in coincidence can be measured. This time difference can be used to calculate the location of the annihilation along a line of response.

In an embodiment, the combined image data can be separated based on a time of flight (TOF) profile of the detected coincidence events. TOF refers to measuring the arrival time difference between the two photons forming the coincidence. Preferably, the detector has a TOF resolution of at least 100 ps to at most 500 ps, more preferably 200 to 400 ps (200 ps = 3 cm Full width at Half maximum). For example, to get sub-centimeter position resolution a timing resolution of less than 100 ps is preferred.

The use of TOF can also enable to separate decays at different locations in image space. As such, by using TOF information, it is possible to acquire the transmission and emission data simultaneously, which means that no extra time is needed for the acquisition of the attenuation data.

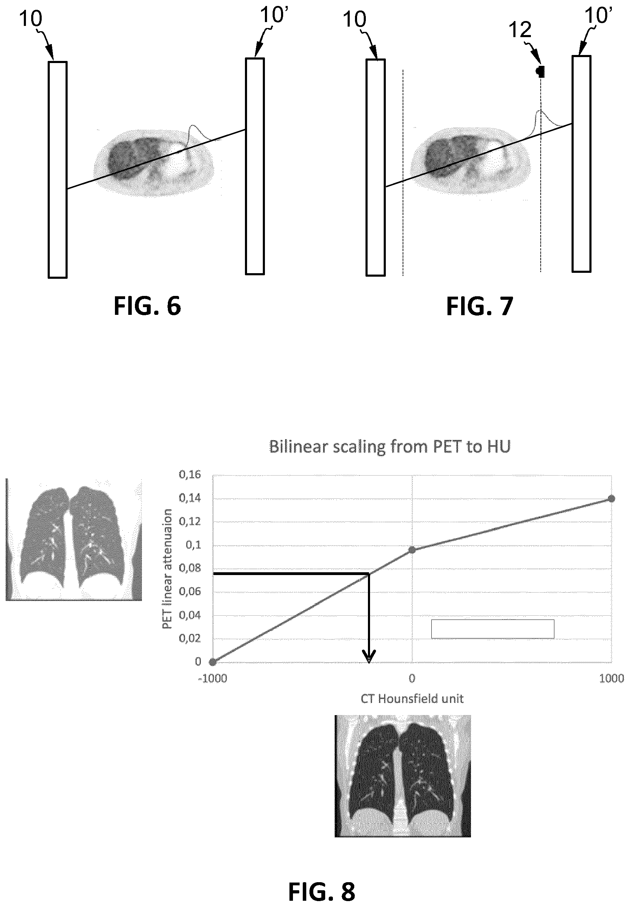

As an example, **Figure 6** shows how photons from an emission coincidence event can be acquired as acquired as emission data. Further, **Figure 7** shows how photons from a transmission coincidence event can be acquired as acquired as transmission data. By comparing **Figure 6** with **Figure 7,** it is clear that photons corresponding to an emission coincidence event have to travel a greater distance than photons corresponding to the coincidence event that is acquired as transmission data. As discussed earlier, coincide events coming different positions in image space can be separated using TOF. The detected coincidence events can be spatially separated by determining the time difference between the detection of two photons forming the coincidence event and determining an emission point based on the time difference, the position of said emission point corresponding to the emission or transmission imaging data. Although a degree of overlap may be expected in the data, this amount can be regarded as negligible compared to the total number of data measured over a complete PET scan.

In another embodiment, the combined image data can be separated based on an energy profile of the detected coincidence events. As previously described, the annihilation of positrons produces two photons of 511 keV each (which is the resting energy of the electron or positron) in opposite directions. In view of this, the detectors in conventional scanners are typically configured to more efficiently absorb the total energy of 511 keV of annihilated photons. This may include the stopping power, amount of light produced against each absorbed photon and time taken for the decay of light. As such, by implementing a radiation source that can generate a transmission image at an energy level different from 511 keV (for example, lower or higher), the coincidence events can be separated based on their respective energy levels, for example, by measuring the energy of each event and then separating the events based on the measured energy levels.

In some embodiment, the combined image data can be separated based on combination of a TOF profile and an energy profile of the detected coincidence events. Combining any of the above embodiments can allow for higher accuracy. Other techniques for the separation of the acquired image data can also be considered. For example, acquired image data may be separated based on positional information of the line of response.

Referring yet again to **Figure 1,** it is shown that once the acquired data has been separated, one or more images can be reconstructed based on the respective data, more specifically, an emission image can be reconstructed from the emission data and a transmission image can be reconstructed from the transmission data. The image(s) can be reconstructed using standard tomographic techniques known in the art. The image quality may be defined based on an image quality parameter, for example, the resolution, the signal-to-noise ratio (SNR) and/or the field of view (FOV). Techniques known in the art may be used to improve the image quality during or after reconstruction. Advantageously, the image quality can be improved by applying a deep learning algorithm.

In some embodiment, an anatomical map can be generated by transforming the transmission image, preferably by applying an inverse bilinear transformation. Images from a CT scan are acquired at lower energies, typically in the range of 50-100 keV. Hence, transmission data obtained at a higher energy, for example 511k EV, transmission measurement can be converted to resembles an image obtained by a lower energy CT scan. An example of this data conversion is shown in **Figure 7.**

In some embodiment, the anatomical map can be generated based on a combination of transmission imaging data with the subject present and with the subject absent (blank data) in the scanning area. Based on this data, a ratio can be made that is preferably logarithmically scaled, for example, by applying the below formula, from which an iterative reconstruction can be performed. Advantageously, the image quality can be improved by applying a deep learning algorithm.

The ratio between the number of detected LORs in a blank scan and a patient scan, can be determined by applying the following formula:

$$I_{out} = I_{in} \, exp[-\int_{x_{in}}^{x_{out}} \mu(x)dx]$$

, such that In

$$\frac{I_{in}}{I_{out}} = \int_{x_{in}}^{x_{out}} \mu(x)dx]$$

. In some embodiments, the deep learning algorithm may comprise a 2D / 3D convolutional neural network (CNN) that has been trained to enhance an image by receiving low resolution and high-resolution image data as input.

The method described herein can generally be performed by or under the control of a processor of a computing unit, for instance, the computing unit of a system described in the present disclosure. The method can be partially or fully automated according to some embodiments thereof. To aid the reader in the understanding of the present disclosure, the method will be explained as being performed by a processor of a medical imaging system. The skilled person will, however, appreciate that the method may be performed by any other processor if appropriately configured, for instance, of a personal computer.

**Claims**

1. System (1) for positron emission tomography (PET) imaging a target volume of a subject injected with a radiotracer, the system comprising

- at least two scanning modules (10), arranged opposite of each other and spaced apart so as to form a scanning area between them; wherein each of the at least two scanning modules comprises:

- at least one PET detector (11) configured to detect the occurrence of a coincidence event in the scanning area; and, - at least one radiation source (12) configured to emit a gamma radiation that illuminates at least one PET detector of the opposite scanning module; and,

- a control unit in communication with the at least two scanning modules, that is configured to:

- acquire combined PET data related to the simultaneous detection of coincidence events from the PET detectors; - spatially separate the combined PET data into transmission data and emission data; - reconstruct a transmission image from the transmission data, wherein the transmission image comprises anatomical information of the target volume; - reconstruct an emission image from the emission data, wherein the emission image comprises molecular information of the radiotracer; and, - generate a combined image based on the transmission image and the emission image.

2. Method for PET imaging a target volume of a subject injected with a radiotracer, the method comprising the steps of:

- providing a PET system comprising at least two scanning modules, arranged opposite of each other, and spaced apart so as to form a scanning area between them; wherein each of the at least two scanning modules comprises at least one PET detector configured to detect the occurrence of a coincidence event in the scanning area, and at least one radiation source configured to emit gamma radiation that illuminates at least one PET detector of the opposite scanning module; - acquiring combined PET data related to the simultaneous detection of coincidence events; - spatially separating the combined PET data into transmission data and emission data; - reconstructing a transmission image from the transmission data, wherein the transmission image comprises anatomical information of the target volume;
- reconstructing an emission image from the emission data, wherein the emission image comprises molecular information of the radiotracer; and
- generating a combined image based on the transmission image and the emission image.

3. The method of claim 2 performed with the system of claim 1.

4. The system and/or method according to any one of the preceding claims, wherein the combined PET data is separated based on a time of flight (TOF) profile of the detected coincidence events; preferably by determining the time difference between detection of two photons forming the detected coincidence event and determining a position of an emission point based on said time difference.

5. The system and/or method according to any one of the preceding claims, wherein the combined PET data is separated based on an energy profile of the detected coincidence events.

6. The system and/or method according to any one of the preceding claims, wherein the transmission data is transformed by applying an inverse bilinear transformation so that the transmission image resembles an image obtained by a lower energy scan, preferably resembles a lower energy computerized tomography (CT) scan.

7. The system and/or method according to any one of the preceding claims, wherein the transmission image reconstructed from the transmission data and a blank data that is acquired by performing a scan with the subject absent.

8. The method according to any one of the preceding claims, wherein the detector module (10) and/or PET detector (11) are an essentially flat panel.

9. The system and/or method according to any one of the preceding claims, wherein a gap between the opposite detector modules (10) is at least 40 cm to at most 100 cm, preferably 40 cm to 80 cm, more preferably 40 cm to 70 cm, more preferably still 45 cm to 60 cm, more preferably still 50 cm to 55 cm.

10. The system and/or method according to any one of the preceding claims, wherein the detector modules (10) are moveably arranged onto a support member so that their position can be adjusted based on a height of the subject.

11. The system and/or method according to any one of the preceding claims, wherein the radiation source (12) is moveably arranged in the detector module (10) so that it can be moved over the PET detector (11) during scanning.

12. The system and/or method according to any one of the preceding claims, wherein the radiation source (12) is spaced apart from the PET detector (11) by a gap of at least 0.1 cm to at most 10.0 cm.

13. The system and/or method according to any one of the preceding claims, wherein the PET detector (11) comprises an array of monolithic scintillation crystals; preferably lutetium-yttrium oxyorthosilicate (LYSO) and/or bismuth germanate (BGO) crystals.

14. The system and/or method according to any one of the preceding claims, wherein the system is a walk-through scanner configured to scan the subject in an upright position.

15. The system and/or method according to any one of the preceding claims, wherein the system comprises a means for positioning the subject in an in in an upright position in the PET scanning area; preferably comprising a handlebar (20) that can be gripped by the subject, a foot stand (21) that the subject can stand on and/or a chair that the subject can sit on.

PERFORM EMISSION SCAN

PERFORM TRANSMISSION SCAN

SIMULTANEOUSLY DETECT COINCIDENCE EVENT(S)

SPATIALLY SEPERATE COMBINED IMAGE DATA

RECONSTRUCT EMISSION IMAGE

RECONSTRUCT TRANSMISSION IMAGE

GENERATE COMBINED IMAGE

**FIG. 1**

**FIG. 2**

**FIG. 3**

**FIG. 4**

**FIG. 5**

**FIG. 6**

**FIG. 7**

Bilinear scaling from PET to HU

**FIG. 8**

Europäisches
Patentamt

European
Patent Office

Office européen
des brevets

# EUROPEAN SEARCH REPORT

Application Number

EP 23 15 1344

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 231 678 B2 (HERRMANN CHRISTOPH [DE]; KONINKLIJKE PHILIPS NV [NL]) 19 March 2019 (2019-03-19) | 1-4,6-9, 12,13 | INV. G01T1/161 G01T1/29 A61B6/03 |
| Y | * column 6, line 6 - line 16 * * figures * * column 6, line 28 - line 52 * * column 3, line 67 * ----- | 10,11, 14,15 | |
| X | US 6 388 244 B1 (GAGNON DANIEL [US]) 14 May 2002 (2002-05-14) | 1-9,12 | |
| Y | * column 1, line 6 - line 13 * * figures * * column 5, line 22 - line 30 * * column 8, line 17 - line 49 * ----- | 10,11, 14,15 | |
| Y | US 7 003 070 B1 (CHEN WILLIAM B [US] ET AL) 21 February 2006 (2006-02-21) * column 2, line 58 - column 3, line 19; figures 1,2 * ----- | 10,14,15 | |
| Y | EP 1 347 309 A2 (HITACHI LTD [JP]) 24 September 2003 (2003-09-24) * figure 1 * ----- | 11 | TECHNICAL FIELDS SEARCHED (IPC) G01T A61B |

The present search report has been drawn up for all claims

| Place of search | Date of completion of the search | Examiner |
|---|---|---|
| The Hague | 24 May 2023 | Eberle, Katja |

EPO FORM 1503 03.82 (P04C01)

## ANNEX TO THE EUROPEAN SEARCH REPORT
## ON EUROPEAN PATENT APPLICATION NO.

EP 23 15 1344

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2023

| Patent document cited in search report | | Publication date | Patent family member(s) | | Publication date |
|---|---|---|---|---|---|
| US 10231678 | B2 | 19-03-2019 | CN | 103220978 A | 24-07-2013 |
| | | | EP | 2640270 A1 | 25-09-2013 |
| | | | RU | 2013127510 A | 27-12-2014 |
| | | | US | 2013237818 A1 | 12-09-2013 |
| | | | WO | 2012066469 A1 | 24-05-2012 |
| US 6388244 | B1 | 14-05-2002 | EP | 1136844 A1 | 26-09-2001 |
| | | | JP | 2002148340 A | 22-05-2002 |
| | | | US | 6388244 B1 | 14-05-2002 |
| US 7003070 | B1 | 21-02-2006 | NONE | | |
| EP 1347309 | A2 | 24-09-2003 | CN | 1445560 A | 01-10-2003 |
| | | | EP | 1347309 A2 | 24-09-2003 |
| | | | KR | 20030076250 A | 26-09-2003 |
| | | | US | 2003179853 A1 | 25-09-2003 |
| | | | US | 2004081277 A1 | 29-04-2004 |
| | | | US | 2004081278 A1 | 29-04-2004 |
| | | | US | 2006188059 A1 | 24-08-2006 |
| | | | US | 2008152074 A1 | 26-06-2008 |

EPO FORM P0459